# EUROPEAN PATENT APPLICATION

(11) **EP 0 870 750 A2**
(43) Date of publication of application: **14.10.1998**
(21) Application number: 98106155.9
(22) Date of filing: 03.04.1998
(51) Int. Cl.: C07C 45/74, C07C 253/30, B01J 21/16

(54) **Catalyst for reactions of the aldol type**

(30) Priority: 07.04.1997 CH 805/97
(71) Applicant: Novartis AG, 4058 Basel (CH); Novartis-Erfindungen Verwaltungsgesellschaft m.b.H., 1235 Wien (AT)
(72) Inventor: Siebenhaar, Bernd, 79400 Kandern (DE); Casagrande, Bruno, 4142 Münchenstein (CH)

(57) **Abstract**

The invention relates to a process for the condensation of aldehydes or ketones with C-H- acidic compounds (especially Knoevenagel reaction) in the presence of a thermally activated fibrous clay mineral of the palygorskite type. The products obtained are important intermediate or end products for the olfactory industry.

## Description

The invention relates to a process for the condensation of aldehydes or ketones (carbonyl component) with C-H- acidic compounds (methylene component) in the presence of a thermally activated fibrous clay mineral of the palygorskite type as catalyst.

The Knoevenagel reaction, in which methylene components with especially high C-H- acidity are used, is a special instance of aldol condensation. The reaction belongs to the standard methods of organic chemistry and is described for example in Principles of Organic Synthesis, R.O.C. Norman, Science Paperbacks 1970, pages 233-234. Basic catalysts are required for the reaction. Those which may be considered are in particular nitrogen bases, especially organic bases such as piperidine, ammonium acetate, β-alanine in glacial acetic acid or also alcoholates. This reaction process has the disadvantage that the resultant reaction water has to be removed, which can be achieved for example with azeotropic distillation by adding toluene. Of course, temperatures of above 100°C are required for this, which is a great disadvantage to temperature-sensitive compounds. During working up of the product, the base must be separated for example by distillation, which frequently is not always achieved quantitatively and can easily lead to coloured products.

The use of NaOH or Na₂CO₃ has similarly been known for a long time and is described e.g. by Bertini in Gazz. Chim. Ital. 31 **l** (1901), 266. One disadvantage here is that it is similarly necessary to eliminate the water, and ester groups that are present may be saponified again under these conditions.

More recently, many attempts have been made to prepare heterogeneous basic catalysts which can be easily separated again after the reaction.

For example, A. Corma et al. in J. Catal. 134 (1992), 58-65, describes the production of a catalyst by the basic modification of hydrotalcite. This production is extremely time-consuming. The resultant gel is dried and calcined several times for 12 or 18 hours. The execution of a Knoevenagel reaction at 130 °C with these catalysts is described.

Another proposal, described in US-A-4.542.002, is based on partially exchanging the Mg²⁺ ions in sepiolites, thus raising the basicity and carrying out the reaction at lower temperatures. In this case also, prior to usage, the catalyst firstly has to be produced by means of a time-consuming ion exchange process.

It has now surprisingly been found that the time-consuming modification in the production of the catalyst can be dispensed with if a thermally activated fibrous clay mineral of the palygorskite type is used as the catalyst. This represents an extremely readily accessible catalyst system with excellent activity. Depending on the reactivity of the methylene component and on the melting point of the two components, aldol reactions may be carried out for example at room temperature. The reaction times are short, there is a high degree of reaction and the reaction is frequently almost quantitative.

Water elimination can be dispensed with, and solvents for the azeotropic distillation do not have to be added. The product quality is very good, since no impurities can ensue through organic bases. Since the catalyst can be separated by simple filtration, working up of the product is considerably simplified.

The catalyst can be immediately re-used after drying, optionally in the reaction receptacle, without noticing a significant loss of activity, even if this cycle is carried out several times. Drying in this instance means that the catalyst is subjected to a temperature of room temperature up to about 250 °C, with or without a vacuum, and/or with or without a current of inert gas.

A further advantage is that the catalyst is also suitable for continuous processes, since it has long standing times.

A first object of the invention is a process for the basically catalysed condensation of an aldehyde or a ketone with a C-H- acidic compound, which is characterised in that the basic catalyst employed is a thermally activated fibrous clay mineral of the palygorskite type.

Thermally activated in terms of the invention means that, after the thermal treatment, the clay material has a residual water content of at most 10% by weight, based on the clay mineral, more preferably at most 5% by weight, and most preferably at most 2% by weight. The thermal treatment may be undertaken in various ways, for example by heating with or without a vacuum, or by heating in a current of inert gas. Heating may indicate temperatures of 200 to 800°C, more preferably 200 to 700°C and most preferably 200 to 600°C. The temperature may be set at a predetermined value from the start, or may be continuously increased starting from room temperature, using a temperature programme. The drying time depends on the respective type of mineral and the heating process, and may range from one hour to 20 hours, preferably from 2 to 10 hours. In the case of straightforward heating, higher temperatures are preferably used, or at lower temperatures treatment takes place over a longer period of time. In the case of thermal treatment in a vacuum or in a current of inert gas, lower temperatures may be used, or also higher temperatures over a shorter period of time.

The aldehyde or the ketone may contain unsubstituted or substituted aliphatic, cycloaliphatic, heteroaliphatic, heterocycloaliphatic, aromatic or heteroaromatic hydrocarbon radicals.

The aliphatic radical includes for example alkyl, especially methyl, ethyl, i-propyl, n-propyl, n-butyl, i-butyl, t-butyl, as well as the different isomeric pentyl, hexyl, heptyl, octyl, nonyl, decyl, undecyl, dodecyl, tridecyl, tetradecyl, pentadecyl, hexadecyl, heptadecyl, octadecyl, nonadecyl and eicosyl radicals.

Alkenyl is also included, for example propenyl, isopropenyl, 2-butenyl, 3-butenyl, isobutenyl, n-penta-2,4-dienyl, 3-methyl-but-2-enyl, n-oct-2-enyl, n-dodec-2-enyl, iso-dodecenyl, n-octadec-2-enyl, n-octadec-4-enyl.

If the aliphatic radical is substituted, this may be hydroxyalkyl, for example hydroxymethyl, hydroxyethyl, 1-hydroxyisopropyl, 1-hydroxy-n-propyl, 2-hydroxy-n-butyl, 1-hydroxy-iso-butyl, 1-hydroxy-secondary-butyl, 1-hydroxy-tertiary-butyl, as well as the different isomeric pentyl, hexyl, heptyl, octyl, nonyl, decyl, undecyl, dodecyl, tridecyl, tetradecyl, pentadecyl, hexadecyl, heptadecyl, octadecyl, nona decyl and eicosyl radicals.

This may also be halogen-alkyl, for example fluoromethyl, difluoromethyl, trifluoromethyl, chloromethyl, dichloromethyl, trichloromethyl, 2,2,2-trifluoroethyl, 2-fluoroethyl, 2-chloroethyl, 2,2,2-trichloroethyl, as well as halogenated, especially fluorinated or chlorinated alkanes, for example the isopropyl, n-propyl, n-butyl, iso-butyl, sec-butyl, tert-butyl radicals and the different isomeric pentyl, hexyl, heptyl, octyl, nonyl, decyl, undecyl, dodecyl, tridecyl, tetradecyl, pentadecyl, hexadecyl, heptadecyl, octadecyl, nona decyl and eicosyl radicals.

Cyanoalkyl includes for example cyanomethyl (methylnitrile), cyanoethyl (ethylnitrile), 1-cyano-isopropyl, 1-cyano-n-propyl, 2-cyano-n-butyl, 1-cyano-iso-butyl, 1-cyano-sec-butyl, 1-cyano-tert-butyl, as well as the different isomeric cyanopentyl and -hexyl radicals.

Heteroaliphatic radical signifies that the aliphatic radical may be interrupted by one or more hetero atoms such as O, S or N, whereby similarly the above-mentioned substituents may appear. In the case of several hetero atoms, the radicals in which the hetero atoms have no direct bonding with one another are preferred.

The cycloalkyl in question preferably concerns C₅-C₈-cycloalkyl, especially C₅- or C₆-cycloalkyl. A few examples are cyclopropyl, dimethylcyclopropyl, cyclobutyl, cyclopentyl, methylcyclopentyl, cyclohexyl, cycloheptyl and cyclooctyl.

The heterocycloalkyl in question concerns radicals with preferably 4 or 5 C-atoms and one or two hetero atoms from the group O, S and N. They may be for example oxirane, azirine, 1,2-oxathiolane, pyrazoline, pyrrolidine, piperidine, piperazine, morpholine, tetrahydrofuran or tetrahydrothiophene.

The aryl in question concerns radicals with preferably 6 to 10 C-atoms. They may be for example phenyl, pentalin, indene, naphthalene, azuline or anthracene.

The heteroaryl in question concerns radicals with preferably 4 or 5 C-atoms and one or two hetero atoms from the group O, S and N. They may be for example pyrrole, furan, thiophene, oxazole, thiazole, pyridine, pyrazine, pyrimidine, pyridazine, indole, purine or quinoline.

Examples of substituents of the aliphatic, cycloaliphatic, heteroaliphatic, heterocycloaliphatic, aromatic or heteroaromatic hydrocarbon radicals or substituted hydrocarbon radicals are: halogen, -CN, -NO₂, R₆R₇R₈Si-(O)ᵤ-, -COOM, -SO₃M, -PO₃M, -COO(M₁)_{1/2}, -SO₃(M₁)_{1/2}, -PO₃(M₁)_{1/2}, C₁-C₂₀-alkyl, C₁-C₂₀-hydroxyalkyl, C₁-C₂₀-halogenalkyl, C₁-C₆-cyanoalkyl, C₃-C₈-cycloalkyl, C₆-C₁₆-aryl, C₇-C₁₆-aralkyl, C₃-C₆-heterocycloalkyl, C₃-C₁₆-heteroaryl, C₄-C₁₆-heteroaralkyl or R₁₄-X₁-; or radicals in which two adjacent C-atoms are optionally substituted by -CO-O-CO- or -CO-NR₁₀-CO-; or in which an alicyclic, aromatic or heteroaromatic ring is condensed on adjacent carbon atoms of the alicyclic ring; whereby X₁ is -O-, -S-, -CO-, -SO-, -SO₂-, -O-C(O)-, -C(O)-O-, -C(O)-NR₁₀-, -NR₁₅-C(O)-, -SO₂-O- or -O-SO₂- ; whereby R₆, R₇ and R₈, independently of one another, signify C₁-C₁₂-alkyl, C₁-C₁₂-perfluoroalkyl, phenyl or benzyl; whereby R₁₄ signifies C₁-C₂₀-alkyl, C₁-C₂₀-halogenalkyl, C₁-C₂₀-hydroxyalkyl, C₃-C₈-cycloalkyl, C₆-C₁₆-aryl, C₇-C₁₆-aralkyl; R₁₀ and R₁₅, independently of one another, signify hydrogen, C₁-C₁₂-alkyl, phenyl or benzyl, whereby the alkyl groups for their part are unsubstituted or substituted by C₁-C₁₂-alkoxy or C₃-C₈-cycloalkyl; whereby M is an alkali metal and M₁ is an alkaline earth metal; and u is 0 or 1.

As the carbonyl component, a compound of formula I 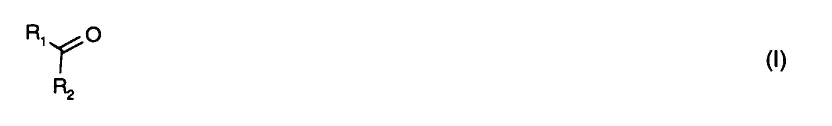 is preferably used, wherein
R₁ and R₂, independently of one another, signify H, C₁-C₁₂-alkyl, phenyl, phenyl-(C₁-C₁₂)-alkyl, or phenyl or phenyl-(C₁-C₁₂)-alkyl substituted once or several times by halogen, C₁-C₄-alkyl, C₁-C₄-alkoxy, -OH or -COOR₃, or R₁ also signifies OH, and
R₃ is H or C₁-C₁₂-alkyl.

Halogen denotes fluorine, chlorine, bromine or iodine.

The C₁-C₁₂-alkyl radicals may be linear or branched, e.g. methyl, ethyl and also the different position isomers of propyl, butyl, pentyl, hexyl, heptyl, octyl, nonyl, decyl, undecyl or dodecyl.

Phenyl-(C₁-C₁₂)-alkyl denotes for example benzyl, phenethyl, 3-phenylpropyl, 4-phenylbutyl, 3-phenylbutyl, 2-phenylbutyl, 5-phenylpentyl, 4-phenylpentyl, 3-phenylpentyl, 2-phenylpentyl or die different position isomers of phenylhexyl, phenylheptyl, phenyloctyl, phenylnonyl, phenyldecyl, phenylundecyl or phenyldodecyl.

The C₁-C₄-alkoxy radicals may be linear or branched, and signify for example methoxy, ethoxy, and also the different position isomers of propoxy or butoxy.

C₁-C₄-alkyl-substituted phenyl includes for example methylphenyl, dimethylphenyl, trimethylphenyl, ethylphenyl, diethylphenyl, isopropylphenyl or tert.-butylphenyl.

The process wherein the carbonyl component is an aldehyde, so that R ₁ is H, is preferred.

R₂ preferably signifies phenyl, phenyl-(C₁-C₁₂)-alkyl, or phenyl or phenyl-(C₁-C₁₂)-alkyl substituted once or several times by halogen, C ₁-C₄-alkyl, C₁-C₄-alkoxy or -OH.

Most preferably, a compound of formula Ia is used as the aldehyde 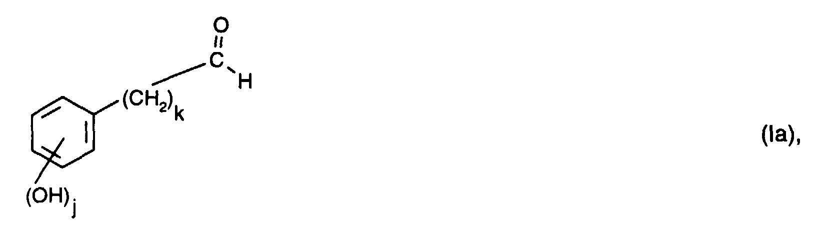
wherein j is 0 or 1 and k signifies 0 or a number from 1 to 6.

Apart from compounds of formula (I), the C-H- acidic compound in question may also be in particular those which have one or two electron-attracting radicals adjacent to a methylene group.

A compound of formula II 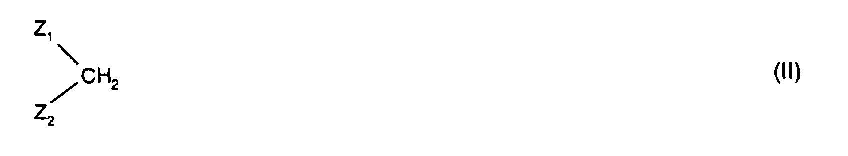 is preferably used as the C-H- acidic compound, wherein
Z₁ and Z₂, independently of one another, signify -COOR₄, -COOH, -CONHR₄, -COR₄, -CN, -SO₂R₄, -SR₄ or quaternated pyridine and
R₄ is C₁-C₁₂-alkyl, or phenyl or naphthyl either unsubstituted or substituted once or several times by halogen, CN, C₁-C₁₂-alkyl or COO(C₁-C₁₂)-alkyl.

Attapulgite and sepiolite belong in particular to the fibrous clay minerals of the palygorskite type. Attapulgite has the idealised composition Mg₅Si₈O₂₀(OH)₂(H₂O)₄. Sepiolite, also known by the name Meerschaum, has the idealised composition Mg₈Si₁₂O₃₀OH₄(H₂O)₄. An overview of structures, properties and technical applications of these compounds is given for example by R.M. Barrer FRS in "Zeolites and Clay Minerals as Sorbents and Molecular Sieves", Academic Press, 1978, page 415/6.

Both naturally occurring and artificially produced fibrous clay minerals of the palygorskite type may be used for the application according to the invention. The fibrous clay minerals of the palygorskite type are available commercially, for example under the Trade Names "ATTAGEL" (Chemie-Mineralien AG & Co KG, Bremen, Germany), "IGS", "SEA MUD", SEPIOGEL A", "SEPIOGEL F", "SEPIOGEL UF", "THERMOGEL", "SEPIOLITE NOODLES", "SEPIOLITE ORE", "WET SEPIOLITE NOODLES", "SEPIOGEL FG", "GRANULAR SEPIOLITE", "SEPIOGEL FLM", "THERMOGEL B" (IMV Division of Floridin Comp., Berkeley Springs, WV, US), "ATTAGEL", "ATTASORB" and "ATTACOTE" (Engelhard Corporation, Edison, NJ, US).

It is preferably to use naturally occurring attapulgite and in particular sepiolite.

Clay minerals of the palygorskite type have the property that the temperature-programmed desorption of chemisorbed CO₂ at normal pressure in the range 100 - 200°C takes place with higher intensity than in the range 450 - 600 °C.
The temperature-programmed desorption of chemisorbed CO₂ at normal pressure is normally carried out as follows:
- a current of carbon dioxide is passed over about 0.5 g of the fibrous clay mineral at a rate of 5 ml/min for 12 hours at room temperature;
- the sample is rinsed for 1 hour with argon (50 ml/min );
- desorption of the CO₂ takes place in a current of argon (50 ml/min) at a heating rate of 10°C/min up to a final temperature of 600 °C;
- detection of the CO ₂ is effected by means of mass spectrometry (M=44).
A similar process is also described in Journal of Catalysis **146**, 155-165 (1994).

It is usual and preferred if no alkali carbonates or alkaline earth carbonates are used as an additional catalyst component apart from the fibrous clay minerals of the palygorskite type.

The fibrous clay minerals of the palygorskite type preferably have a specific surface area greater than 75 m²/g.

In order to obtain the catalyst according to the invention, the fibrous clay mineral of the palygorskite type is usually treated thermally at elevated temperatures. Of practical significance are temperatures of 200 to 800°C for preferably 3 to 8 hours. After the thermal treatment, the residual water content is generally less than 10, preferably less than 5 and most preferably less than 2% by weight. The clay mineral usually exists in powder form or pellet form.

The fibrous clay minerals of the palygorskite type are preferably used in a quantity of 5 to 50 g, most preferably 10 to 20 g per mol of carbonyl component.

The process is preferably carried out in batch operation at a temperature of 20 to 150°C, most preferably at a temperature of 50 to 90 °C.

The process is preferably carried out in continuous operation at a temperature of 100 to 300°C, most preferably at a temperature of 120 to 190 °C.

The reaction can be carried out with or without solvents. If low-melting or liquid starting products are used, preferably no solvent is added. If solvents are used, toluene, xylene or an aliphatic hydrocarbon are e.g. particularly suitable.

The reaction is normally carried out without inert gas and under normal pressure conditions.

A further object of the invention is a thermally activated fibrous clay mineral of the palygorskite type, which preferably has a residual water content of less than 10% by weight, and the use of the catalyst for the basically catalysed condensation of aldehydes or ketones with C-H- acidic compounds. The above-described significances and preferences for the process similarly apply to the catalyst and its usage.

The products obtained are important intermediate or end products for the olfactory industry. DE-OS 2 256 483 names a series of olfactory and aromatic substances by way of example.

The following examples illustrate the invention.

### A) Preparation of a catalyst

Example A: 50 g of a commercial sepiolite (e.g. Florex 725 i) or attapulgite (Attasorb) are slowly heated to 550°C under a normal atmosphere in an annealing furnace. This temperature is maintained for 5 hours. The catalyst is filled into a sealed container (atmospheric moisture) whilst still hot (approximately 200 °C) and can be stored in this way for several weeks.

Example B: For usage in the continuous process, the sepiolite or attapulgite may be treated thermally directly before the reaction. To do so, 5 ml of the catalyst are heated to 550°C in the reactor and in a current of air (20 ml/min). This temperature is similarly maintained for 5 hours.

Afterwards, there is cooling to reaction temperature in a current or air or protective gas. Upon reaching this temperature, the continuous reaction can be started immediately.

### B) Preparation of compounds

Example 1: 0.1 mols respectively of benzaldehyde (10.61 g) and cyanoacetic acid ethyl ester (11.31 g) are placed in a flask which has a reflux condenser, and heated to 55°C. Then, 2.2 g of sepiolite are added (annealed for 5 hours at 550°C). The reaction commences instantly and is complete after 40 minutes. The reaction mixture is filtered off whilst still hot. The product ,cyano-cinnamic acid ethyl ester, precipitates already in the washing bottle in a purity of > 96%. The yield is similarly > 96%.

Example 2: 0.1 mols respectively of benzaldehyde (10.61 g) and cyanoacetic acid ethyl ester (11.31 g) are placed in a flask which has a reflux condenser, and heated to 55°C. Then, 2.2 g of attapulgite are added (annealed for 5 hours at 600°C). The reaction commences instantly and is complete after 55 minutes. The reaction mixture is filtered off whilst still hot. The product, cyano-cinnamic acid ethyl ester, precipitates already in the washing bottle in a purity of > 95%. The yield is > 96%.

Working up variant for examples 1 and 2:
The still hot reaction mixture is added to 200 ml of ethanol, heated to 50 - 60°C and filtered off. Upon cooling, the cyano-cinnamic acid ethyl ester crystallises in a purity of over 99%. The yield is 95%.

Example 3: A mixture of 1 mol of benzaldehyde and 1 mol of cyanoacetic acid ethyl ester is passed at 2 ml/hour over 5 ml of sepiolite catalyst (annealed for 5 hours at 550°C). The temperature of the catalyst bed is 120°C. The product, cyano-cinnamic acid ethyl ester, is collected in a trap which is cooled with water. The yield is > 92%, and the selectivity for cyano-cinnamic acid ethyl ester is 96%. After 120 hours 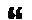 on stream 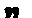 , the catalyst shows hardly any disactivation, but the selectivity even increases by 1-2%.

Example 4: A mixture of 1 mol of benzaldehyde and 1 mol of malonic acid diethyl ester is passed at 2 ml/hour over 5 ml of sepiolite catalyst (annealed for 5 hours at 550°). The temperature of the catalyst bed is 200°C. The product is collected in a trap which is cooled with water. The yield of benzylidene malonic acid diethyl ester of 48%, and the selectivity for cyano-cinnamic acid ethyl ester is 98%.

## Claims

1. Process for the basically catalysed condensation of an aldehyde or ketone with a C-H- acidic compound, characterised in that a thermally activated fibrous clay mineral of the palygorskite type is used as the basic catalyst.

2. Process according to claim 1, characterised in that the carbonyl component is a compound of formula I 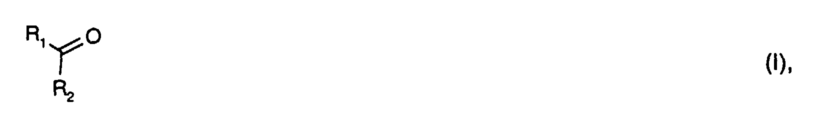 wherein
R₁ and R₂, independently of one another, signify H, C₁-C₁₂-alkyl, phenyl, phenyl-(C₁-C₁₂)-alkyl, or phenyl or phenyl-(C₁-C₁₂)-alkyl substituted once or several times by halogen, C₁-C₄-alkyl, C₁-C₄-alkoxy, -OH or -COOR₃, or R₁ also signifies OH, and
R₃ is H or C₁-C₁₂-alkyl.

3. Process according to claim 1, characterised in that the carbonyl component is an aldehyde.

4. Process according to claim 2, characterised in that R₁ is H and R₂ signifies phenyl, phenyl-(C₁-C₁₂)-alkyl, or phenyl or phenyl-(C₁-C₁₂)-alkyl substituted once or several times by halogen, C₁-C₄-alkyl, C₁-C₄-alkoxy or -OH.

5. Process according to claim 1, characterised in that a compound of formula II 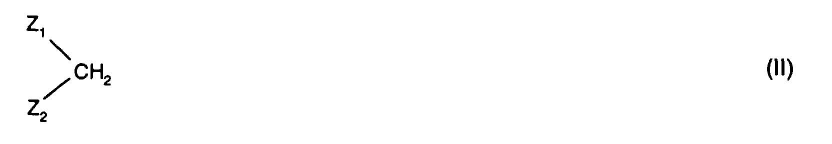 is used as the C-H- acidic compound, wherein
Z₁ and Z₂, independently of one another, signify -COOR₄, -COOH, -CONHR₄, -COR₄, -CN, -SO₂R₄, -SR₄ or quaternated pyridine and
R₄ is C₁-C₁₂-alkyl, or phenyl or naphthyl either unsubstituted or substituted once or several times by halogen, CN, C₁-C₁₂-alkyl or COO(C₁-C₁₂)-alkyl.

6. Process according to claim 1, characterised in that the fibrous clay mineral of the palygorskite type has a specific surface area greater than 75 m ²/g.

7. Process according to claim 1, characterised in that sepiolite or attapulgite is used as the fibrous clay mineral of the palygorskite type.

8. Process according to claim 1, characterised in that the dried fibrous clay mineral has a residual water content of at most 10% by weight, based on the clay mineral.

9. Process according to claim 1, characterised in that the fibrous clay mineral of the palygorskite type is used in a quantity of 5 to 50 g per mol of carbonyl component.

10. Process according to claim 1, characterised in that it is carried out in a batch operation at a temperature of 20 to 150°C, or in the case of continuous operation at a temperature of 100 to 300°C.

11. Process according to claim 1, characterised in that subsequently to the condensation reaction, the catalyst is separated, dried and re-used.

12. A thermally activated fibrous clay mineral of the palygorskite type.

13. Use of a clay mineral according to claim 12 as a catalyst for the basically catalysed condensation of aldehydes and ketones with compounds which have an activated methylene group.
